(19) Europäisches Patentamt
European Patent Office
Office européen des brevets

(11) **EP 2 653 155 A1**

(12) **EUROPEAN PATENT APPLICATION**
published in accordance with Art. 153(4) EPC

(43) Date of publication:
**23.10.2013 Bulletin 2013/43**

(21) Application number: **11848961.6**

(22) Date of filing: **15.12.2011**

(51) Int Cl.:
*A61K 9/08* (2006.01)     *A61K 31/045* (2006.01)
*A61K 31/122* (2006.01)     *A61K 31/765* (2006.01)
*A61L 2/18* (2006.01)     *A61P 31/04* (2006.01)
*G02C 13/00* (2006.01)

(86) International application number:
**PCT/JP2011/079064**

(87) International publication number:
**WO 2012/081673 (21.06.2012 Gazette 2012/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.12.2010   JP 2010281783**

(71) Applicant: **Rohto Pharmaceutical Co., Ltd.
Osaka-shi
Osaka 544-8666 (JP)**

(72) Inventors:
• **FURUMIYA, Chinatsu
Osaka-shi, Osaka 544-8666 (JP)**
• **NAKATA, Atsuko
Osaka-shi, Osaka 544-8666 (JP)**

(74) Representative: **HOFFMANN EITLE
Patent- und Rechtsanwälte
Arabellastrasse 4
81925 München (DE)**

(54) **OPHTHALMIC COMPOSITION FOR CONTACT LENS**

(57)     An object of the present invention is to provide an ophthalmic composition for contact lens that can inhibit bacterial adhesion to a contact lens.

The ophthalmic composition for contact lens can be prepared by using a combination of (A) polyoxyethylene castor oil and (B) terpenoid.

EP 2 653 155 A1

**Description**

Technical Field

The present invention relates to a contact lens composition that can inhibit bacterial adhesion to a contact lens. The present invention also relates to a method for inhibiting bacterial adhesion to a contact lens. The present invention further relates to a method for inhibiting lipid adhesion to a contact lens.

Background Art

The number of people wearing contact lenses has increased in recent years. The clean state of contact lenses, which are used in direct contact with an ocular mucous membrane, must be maintained as much as possible; thus, reducing the adhesion of foreign matter such as bacteria during use is important. Many normal (indigenous) bacteria on the conjunctiva are non-pathogenic bacteria; however, excessive adhesion of bacteria to contact lenses allows extracellular material, etc., secreted from the adhered bacteria to form a biofilm on the contact lens surface. The biofilm may be at risk for becoming a hotbed for pathogenic microorganisms, increasing the risk of microorganism infection.
A contact lens carrying an antimicrobial substance has been reported as a known technique for inhibiting bacterial adhesion to a contact lens (Patent Literature 1 and 2). However, the technique for inhibiting bacterial adhesion to a contact lens has not been fully examined from the viewpoint of the formulation design of ophthalmic compositions for contact lens, such as eye drops, eye washes, and contact lens care solutions.
Polyoxyethylene castor oil, which is a nonionic surfactant, is conventionally used in ophthalmic compositions to help dissolve ingredients (see Patent Literature 3), and is one of the effective solubilizing agents.
Terpenoid is used in ophthalmologic compositions in order to provide a cooling sensation during application (see Patent Literature 4).
However, the effects of polyoxyethylene castor oil or terpenoid on bacterial adhesion to a contact lens have not been found; naturally, the effects of the combination use of polyoxyethylene castor oil and terpenoid on bacterial adhesion to a contact lens have also not been found.

Citation List

Patent Literature

     PTL 1: JP2003-248200A
     PTL 2: JP2009-533081A
     PTL 3: JP2005-298448A
     PTL 4: JP2004-315517A

Summary of Invention

Technical Problem

An object of the present invention is to provide an ophthalmic composition for contact lens, the composition being capable of inhibiting bacterial adhesion to a contact lens.

Solution to Problem

The present inventors conducted extensive research to solve the above problem. As a result, they surprisingly found that bacterial adhesion to a contact lens is effectively  inhibited in an ophthalmic composition for contact lens comprising terpenoid together with polyoxyethylene castor oil. Further research by the present inventors unexpectedly found that the combination use of polyoxyethylene castor oil and terpenoid can inhibit lipid adsorption to a contact lens.
The present invention was accomplished as a result of further research based on these findings.
The present invention provides ophthalmic compositions according to the following embodiments.
Item 1-1. An ophthalmic composition for contact lens, comprising (A) polyoxyethylene castor oil and (B) terpenoid.
Item 1-2. The ophthalmic composition for contact lens according to Item 1-1, wherein the content of component (A) is 0.01 to 3 w/v% based on the total amount of the ophthalmic composition for contact lens.
Item 1-3. The ophthalmic composition for contact lens according to Item 1-1 or 1-2, wherein the polyoxyethylene castor oil is polyoxyethylene castor oil having an average number of moles of added ethylene oxide of 3 to 60 (e.g., polyoxyethylene castor oil 3, polyoxyethylene castor oil 10, polyoxyethylene castor oil 20, polyoxyethylene castor oil 35, polyoxyethylene castor oil 40, polyoxyethylene castor oil 50, and polyoxyethylene castor oil 60).

Item 1-4. The ophthalmic composition for contact lens according to any one of Items 1-1 to 1-3, wherein component (B) is at least one member selected from the group consisting of menthol, camphor, geraniol, borneol, and cineol.

Item 1-5. The ophthalmic composition for contact lens according to any one of Items 1-1 to 1-4, wherein the content of component (B) is 0.0001 to 1 w/v% based on the total amount of the ophthalmic composition for contact lens.

Item 1-6. The ophthalmic composition for contact lens according to one of Items 1-5, wherein the total amount of component (B) is 0.01 to 1,000 parts by weight per 100 parts by weight of the total amount of component (A).

Item 1-7. The ophthalmic composition for contact lens according to any one of Items 1-1 to 1-6, which further comprises a buffer.

Item 1-8. The ophthalmic composition for contact lens according to Item 1-7, wherein the buffer is a boric acid buffer.

Item 1-9. The ophthalmic composition for contact lens according to Item 1-7 or 1-8, wherein the content of the buffer is 0.01 to 10 w/v% based on the total amount of the ophthalmic composition for contact lens.

Item 1-10. The ophthalmic composition for contact lens according to any one of Items 1-1 to 1-9, which further comprises a nonionic surfactant other than polyoxyethylene castor oil.

Item 1-11. The ophthalmic composition for contact lens according to Item 1-10, wherein the nonionic surfactant other than polyoxyethylene castor oil is at least one member selected from the group consisting of polyoxyethylene sorbitan fatty acid esters, polyoxyethylene hydrogenated castor oils, and polyoxyethylene polyoxypropylene block copolymers.

Item 1-12. The ophthalmic composition for contact lens according to Item 1-10 or 1-11, wherein the content of the nonionic surfactant other than polyoxyethylene castor oil is 0.001 to 3 w/v% based on the total amount of the ophthalmic composition for contact lens.

Item 1-13. The ophthalmic composition for contact lens according to any one of Items 1-1 to 1-12, which is an eye drops for contact lens.

Item 1-14. The ophthalmic composition for contact lens according to any one of Items 1-1 to 1-12, which is an eye wash for contact lens.

Item 1-15. The ophthalmic composition for contact lens according to any one of Items 1-1 to 1-12, which is a solution for wearing a contact lens.

Item 1-16. The ophthalmic composition for contact lens according to any one of Items 1-1 to 1-12, which is a contact lens care solution.

Item 1-17. The ophthalmic composition for contact lens according to any one of Items 1-1 to 1-12, which is a disinfectant, cleansing, and storage solution for contact lens.

Item 1-18. The ophthalmic composition for contact lens according to any one of Items 1-1 to 1-17, wherein the contact lens is a hard contact lens.

Item 1-19. The ophthalmic composition for contact lens according to any one of Items 1-1 to 1-17, wherein the contact lens is an oxygen-permeable hard contact lens.

Item 1-20. The ophthalmic composition for contact lens according to any one of Items 1-1 to 1-17, wherein the contact lens is a soft contact lens.

Item 1-21. The ophthalmic composition for contact lens according to any one of Items 1-1 to 1-17, wherein the contact lens is a silicone hydrogel contact lens.

The present invention provides methods for inhibiting bacterial adhesion to a contact lens, methods for providing an effect of inhibiting bacterial adhesion to a contact lens, methods for inhibiting lipid adsorption to a contact lens, methods for providing an effect of inhibiting lipid adsorption to a contact lens, and methods for producing an ophthalmic composition for contact lens according to the following embodiments.

Item 2. A method for inhibiting bacterial adhesion to a contact lens, comprising bringing an ophthalmic composition for contact lens containing (A) polyoxyethylene castor oil and (B) terpenoid into contact with the contact lens.

Item 3. A method for providing an ophthalmic composition for contact lens with an effect of inhibiting bacterial adhesion to a contact lens, comprising adding (A) polyoxyethylene castor oil and (B) terpenoid to the ophthalmic composition for contact lens.

Item 4. A method for inhibiting lipid adsorption to a contact lens, comprising bringing an ophthalmic composition for contact lens containing (A) polyoxyethylene castor oil and (B) terpenoid into contact with the contact lens.

Item 5. A method for providing an ophthalmic composition for contact lens with an effect of inhibiting lipid adsorption to a contact lens, comprising adding (A) polyoxyethylene castor oil and (B) terpenoid to the ophthalmic composition for contact lens.

Item 6. A method for producing an ophthalmic composition for contact lens having an effect of inhibiting bacterial adhesion and/or an effect of inhibiting lipid adsorption to a contact lens, comprising adding (A) polyoxyethylene castor oil and (B) terpenoid to a carrier selected from water and water-containing ethanol.

Item 7. The method according to any one of Items 2 to 6, wherein the polyoxyethylene castor oil is polyoxyethylene castor oil having an average number of moles of added ethylene oxide of 3 to 60 (e.g., polyoxyethylene castor oil 3, polyoxyethylene castor oil 10, polyoxyethylene castor oil 20, polyoxyethylene castor oil 35, polyoxyethylene castor oil 40, polyoxyethylene castor oil 50, and polyoxyethylene castor oil 60).

Item 8. The method according to any one of Items 2 to 7, wherein component (B) is at least one member selected from the group consisting of menthol, camphor, geraniol, borneol, and cineol.

Item 9. The method according to any one of Items 2 to 8, wherein the content of component (B) in the ophthalmic composition for contact lens is 0.0001 to 1 w/v% based on the total amount of the ophthalmic composition for contact lens.

Item 10. The method according to any one of Items 2 to 9, wherein the total amount of component (B) is 0.01 to 1,000 parts by weight per 100 parts by weight of the total amount of component (A).

Item 11. The method according to any one of Items 2 to 10, wherein the ophthalmic composition for contact lens further comprises a buffer.

Item 12. The method according to Item 11, wherein the buffer is a boric acid buffer.

Item 13. The method according to Item 11 or 12, wherein the content of the buffer in the ophthalmic composition for contact lens is 0.01 to 10 w/v% based on the total amount of the ophthalmic composition for contact lens.

Item 14. The method according to any one of Items 2 to 13, wherein the composition further contains a nonionic surfactant other than polyoxyethylene castor oil.

Item 15. The method according to Item 14, wherein the nonionic surfactant other than polyoxyethylene castor oil is at least one member selected from the group consisting of polyoxyethylene sorbitan fatty acid esters, polyoxyethylene hydrogenated castor oils, and polyoxyethylene polyoxypropylene block copolymers.

Item 16. The method according to Item 14 or 15, wherein the content of the nonionic surfactant other than polyoxyethylene castor oil in the ophthalmic composition for contact lens is 0.001 to 3 w/v% based on the total amount of the ophthalmic composition for contact lens.

Item 17. The method according to any one of Items 2 to 16, wherein the ophthalmic composition for contact lens is an eye drops for contact lens.

Item 18. The method according to any one of Items 2 to 16, wherein the ophthalmic composition for contact lens is an eye wash for contact lens.

Item 19. The method according to any one of Items 2 to 16, wherein the ophthalmic composition for contact lens is a solution for wearing a contact lens.

Item 20. The method according to any one of Items 4 to 16, wherein the ophthalmic composition for contact lens is a contact lens care solution.

Item 21. The method according to any one of Items 2 to 16, wherein the ophthalmic composition for contact lens is a disinfectant, cleansing, and storage solution for contact lens.

Item 22. The method according to any one of Items 2 to 21, wherein the contact lens is a hard contact lens.

Item 23. The method according to any one of Items 2 to 21, wherein the contact lens is an oxygen-permeable hard contact lens.

Item 24. The method according to any one of Items 2 to 21, wherein the contact lens is a soft contact lens.

Item 25. The method according to any one of Items 2 to 21, wherein the contact lens is a silicone hydrogel contact lens.

Advantageous Effects of Invention

According to the present invention, the combination use of polyoxyethylene castor oil and terpenoid can inhibit bacterial adhesion to a contact lens, reducing the risk of bacterial infectious diseases caused by the wearing of a contact lens. Further, the combination use inhibits the formation of a biofilm, which is formed by extracellular material, or the like, secreted from bacteria; and/or adhesion of pathogenic microorganisms, which are generated on the biofilm. That is, the present invention can reduce the risk of pathogenic microorganism infection diseases caused by the wearing of a contact lens. Thus, the present invention provides an ophthalmic composition for contact lens that makes it possible to use a contact lens in a safer manner.

The combination use of polyoxyethylene castor oil and terpenoid can inhibit lipid adsorption (e.g., lipid derived from bodily secretions, cosmetics, etc.) to a contact lens, and reduce lipid deposits on a lens caused by the wearing of the contact lens. Further, the combination use thereof further inhibits discomfort such as blurry vision or a foreign body sensation due to lipid deposits, and the development of microorganisms caused by lipid deposits. Thus, the present invention provides an ophthalmic composition for contact lens that makes it possible to use a contact lens in a safer or more comfortable manner.

Description of Embodiments

In the present specification, the unit of content "%" indicates w/v%, and is the same as g/100 mL.

In the present specification, the abbreviation "POE" means polyoxyethylene, unless otherwise specified.

In the present specification, the abbreviation "POP" means polyoxypropylene, unless otherwise specified.

In the present specification, the abbreviation "SCL" means a soft contact lens, unless otherwise specified.

1. Ophthalmic Composition for Contact Lens

The ophthalmic composition for contact lens of the present invention contains polyoxyethylene castor oil (hereinbelow sometimes referred to as component (A)).

Polyoxyethylene castor oil is a known compound obtained by addition polymerization of ethylene oxide with castor oil, and several kinds of polyoxyethylene castor oils having a different average number of moles of added ethylene oxide are known. The average number of moles of added ethylene oxide in polyoxyethylene castor oil is not particularly limited; however, for example, it is 3 to 60. Specific examples thereof include polyoxyethylene castor oil 3, polyoxyethylene castor oil 10, polyoxyethylene castor oil 20, polyoxyethylene castor oil 35, polyoxyethylene castor oil 40, polyoxyethylene castor oil 50, and polyoxyethylene castor oil 60.

These polyoxyethylene castor oils may be used singly, or in a combination of two or more; and preferably in a combination of two or more. Note that the polyoxyethylene castor oil used in the present invention is a compound that is different from and can be distinguished from polyoxyethylene hydrogenated castor oil obtained by addition polymerization of ethylene oxide with hydrogenated castor oil.

The content of component (A) in the ophthalmic composition for contact lens of the present invention is suitably determined according to the kind of component (A), the kind of component (B) used in combination with component (A), the preparation form of the ophthalmic composition, etc. For example, the total amount of component (A) is 0.01 to 3 w/v%, preferably 0.02 to 2 w/v%, more preferably 0.05 to 1 w/v%, and even more preferably 0.2 to 0.6 w/v%, based on the total amount of the ophthalmic composition for contact lens.

The aforementioned content of component (A) is preferable to further increase an effect of inhibiting bacterial adhesion to a contact lens, and to increase an effect of inhibiting lipid adsorption to a contact lens.

The ophthalmic composition of the present invention contains terpenoid (referred to as component (B)) in addition to component (A). Such a combination use of components (A) and (B) can significantly inhibit bacterial adhesion to a contact lens. The combination use of components (A) and (B) can also significantly inhibit lipid adsorption to a contact lens.

The terpenoid used as component (B) is not particularly limited, as long as it is pharmacologically (pharmaceutically) or physiologically acceptable in the field of medicine. Examples of the terpenoid include menthol, camphor, borneol, geraniol, cineol, citronellol, menthone, carvone, anethole, eugenol, limonene, linalool, lineally acetate, and derivatives thereof. These compounds may be in the d form, 1 form, or dl form. Essential oil containing the above compounds can be used as terpenoid in the present invention. Examples of such essential oil include eucalyptus oil, bergamot oil, peppermint oil, cool mint oil, spearmint oil, mint oil, fennel oil, cinnamon oil, rose oil, and the like. For example, when cineol is used as the terpenoid, eucalyptus oil can be used as a source of cineol. These terpenoids can be used singly, or in a combination of two or more.

Of these components (B), menthol, camphor, geraniol, borneol, cineol, etc., are preferable to further improve an effect of inhibiting bacterial adhesion to a contact lens, and/or an effect of inhibiting lipid adsorption to a contact lens. Preferable examples of essential oil containing the above components include eucalyptus oil, cool mint oil, peppermint oil, mint oil, camphor oil, and the like. Menthol, camphor, geraniol, borneol, and cineol are more preferable, and 1-menthol, d-camphor, dl-camphor, d-borneol, and cineol are even more preferable.

To further increase the inhibition effect of bacterial adhesion to a contact lens and/or the inhibition effect of lipid absorption to a contact lens, the content of component (B) in the ophthalmic composition for contact lens of the present invention is 0.0001 to 1 w/v%, preferably 0.001 to 0.1 w/v%, more preferably 0.003 to 0.06 w/v%, and particularly preferably 0.003 to 0.02 w/v%, based on the total amount of the ophthalmic composition for contact lens. When essential oil containing terpenoid is used as component (B), the total amount of terpenoid in the essential oil to be added is determined such that the aforementioned content is satisfied.

The ratio of component (B) to component (A) in the ophthalmic composition of the present invention is not particularly limited, as long as the aforementioned content is satisfied. To increasingly improve the inhibition effect of bacterial adhesion to a contact lens and/or the inhibition effect of lipid absorption to a contact lens, it is desirable to satisfy the ratio such that the total amount of component (B) is 0.01 to 1,000 parts by weight, preferably 0.1 to 500 parts by weight, more preferably 0.5 to 100 parts by weight, even more preferably 0.5 to 80 parts by weight, particularly preferably 0.5 to 50 parts by weight, and most preferably 1 to 25 parts by weight per 100 parts by weight of the total amount of component (A).

The ophthalmic composition for contact lens of the present invention preferably includes a buffer, by which the pH of the ophthalmic composition of the present invention can be adjusted. A buffer that can be contained in the ophthalmic composition for contact lens of the present invention is not particularly limited, as long as it is a pharmacologically (pharmaceutically) or physiologically acceptable buffer in the field of medicine. Examples of such a buffer include boric acid buffers, phosphoric acid buffers, carbonic acid buffers, citric acid buffers, acetic acid buffers, aspartic acid, aspartic acid salts, and the like. These buffers can be used in combination. Examples of boric acid buffers include boric acid or boric acid salts such as alkali metal borate and alkaline earth metal borate. Examples of phosphoric acid buffers include phosphoric acid or phosphoric acid salts such as alkali metal phosphate and alkaline earth metal phosphate. Examples of carbonic acid buffers include carbonic acid or carbonic acid salts such as alkali metal carbonate and alkaline earth metal carbonate. Examples of citric acid buffers include citric acid or alkali metal citrate, alkaline earth metal citrate, and the like. As a boric acid buffer or a phosphoric acid buffer, a hydrate of boric acid or a hydrate of phosphoric acid can also be used. More specifically, examples of the boric acid buffers include boric acid or salts thereof (sodium borate, potassium tetraborate, potassium metaborate, ammonium borate, borax, etc.); examples of the phosphoric acid buffer include phosphoric acid or salts thereof (disodium hydrogen phosphate, sodium dihydrogenphosphate, potassium dihydrogenphosphate, trisodium phosphate, dipotassium phosphate, calcium monohydrogen phosphate, calcium dihydrogenphosphate, etc.); examples of the carbonic acid buffer include carbonic acid or salts thereof (sodium bicarbonate, sodium carbonate, ammonium carbonate, potassium carbonate, calcium carbonate, potassium bicarbonate, magnesium carbonate, etc.); examples of the citric acid buffer include citric acid or salts thereof (sodium citrate, potassium citrate, calcium citrate, sodium dihydrogen citrate, disodium citrate, etc.); and examples of the acetic acid buffer include acetic acid or salts thereof (ammonium acetate, potassium acetate, calcium acetate, sodium acetate, etc.), and aspartic acid or salts thereof (sodium aspartate, magnesium aspartate, potassium aspartate, etc.). Of the buffers, a boric acid buffer (especially, a combination of boric acid and borax) is preferable.

When the ophthalmic composition for contact lens of the present invention includes the buffer, the content of the buffer cannot be uniformly determined, and varies depending on the kind of buffer to be used, the kinds and contents of other components, the preparation form of the ophthalmic composition for contact lens, etc. For example, the total amount of the buffer is 0.01 to 10 w/v%, preferably 0.05 to 5 w/v%, more preferably 0.1 to 2.5 w/v%, and even more preferably 0.1 to 1 w/v%, based on the total amount of the ophthalmic composition for contact lens.

The ophthalmic composition for contact lens of the present invention further includes a surfactant other than polyoxyethylene castor oil. Such a surfactant is not particularly limited, as long as it is pharmacologically (pharmaceutically) or physiologically acceptable in the field of medicine. The surfactant may be a nonionic surfactant, ampholytic surfactant, anionic surfactant, or cationic surfactant.

Specific examples of the nonionic surfactant that may be contained in the ophthalmic composition for contact lens of the present invention include POE sorbitan fatty acid esters such as POE (20) sorbitan monolaurate (polysorbate 20), POE (20) sorbitan monopalmitate (polysorbate 40), POE (20) sorbitan monostearate (polysorbate 60), POE (20) sorbitan tristearate (polysorbate 65), and POE (20) sorbitan monooleate (polysorbate 80); POE hydrogenated castor oils such as POE (60) hydrogenated castor oil (polyoxyethylene hydrogenated castor oil 60); POE alkyl ethers such as POE (9) lauryl ether; POE-POP alkyl ethers such as POE (20) POP(4) cetyl ether; polyoxyethylene-polyoxypropylene block copolymers such as POE (196) POP (67) glycol (poloxamer 407 and pluronic F127), and POE (200) POP (70) glycol. In the compounds listed above, POE means polyoxyethylene, POP means polyoxypropylene, and each of the numbers in the parentheses shows the addition mole number. Further, specific examples of the ampholytic surfactant that can be contained in the ophthalmic composition for contact lens of the present invention include alkyldiaminoethylglycine or salts thereof (e.g., hydrochloride).

Further, specific examples of the cationic surfactant that can be contained in the ophthalmic composition for contact lens of the present invention include benzalkonium chloride, benzethonium chloride, and the like.

Specific examples of the anionic surfactant that can be contained in the ophthalmic composition for contact lens of the present invention include alkylbenzene sulfonate, alkyl sulfate, polyoxyethylene alkyl sulfate, aliphatic $\alpha$-sulfomethyl ester, $\alpha$-olefin sulfonic acid, and the like. Nonionic surfactants are preferable, and POE sorbitan fatty acid esters, POE hydrogenated castor oils, and POE-POP block copolymers are more preferable. Polysorbate 80, polyoxyethylene hydrogenated castor oil 60, and poloxamer 407 are particularly preferable.

Of these surfactants, nonionic surfactants are preferable.

In the ophthalmic composition for contact lens of the present invention, the surfactants can be used singly, or in a combination of two or more.

When the ophthalmic composition for contact lens of the present invention includes the surfactant, the content of the surfactant may be suitably determined according to the kind of surfactant, the kinds and the contents of other components, the preparation form of the ophthalmic composition for contact lens, etc. As an example of the content of the surfactant, the total amount of the surfactant is 0.001 to 3 w/v%, preferably 0.01 to 2 w/v%, more preferably 0.05 to 1 w/v%, and particularly preferably 0.1 to 1 w/v%, based on the total amount of the ophthalmic composition for contact lens.

The ophthalmic composition for contact lens of the present invention may further include a tonicity agent. The tonicity agent that can be contained in the ophthalmic composition for contact lens of the present invention is not particularly limited, as long as it is pharmacologically (pharmaceutically) or physiologically acceptable in the field of medicine. Examples of the tonicity agent include disodium hydrogen phosphate, sodium dihydrogenphosphate, potassium dihydrogenphosphate, sodium hydrogen sulfite, sodium sulfite, potassium chloride, calcium chloride, sodium chloride, magnesium chloride, potassium acetate, sodium acetate, sodium bicarbonate, sodium carbonate, sodium thiosulfate, magnesium sulfate, glycerin, propylene glycol, and the like. Of these tonicity agents, preferable examples include glycerin, propylene glycol, sodium chloride, potassium chloride, calcium chloride, and magnesium chloride, more preferable examples include sodium chloride and glycerin, and particularly preferable examples include sodium chloride. These tonicity agents can be used singly, or in a combination of two or more.

When the ophthalmic composition for contact lens of the present invention includes a tonicity agent, the content of the tonicity agent cannot be limited, and it varies depending on the kind of tonicity agent to be used. For example, the total amount of the tonicity agent is 0.01 to 10 w/v%, preferably 0.05 to 5 w/v%, and more preferably 0.1 to 3 w/v%, based on the total amount of the ophthalmic composition for contact lens.

The pH of the ophthalmic composition for contact lens of the present invention is not particularly limited, as long as it is within a pharmacologically (pharmaceutically) or physiologically acceptable range in the field of medicine. The pH of the ophthalmic composition for contact lens of the present invention is within the range of, for example, 4.0 to 9.5, preferably 5.0 to 9.0, more preferably 6.2 to 8.5, even more preferably 6.5 to 8, and particularly preferably about 6.5 to 7.5.

The osmotic pressure of the ophthalmic composition for contact lens of the present invention is not particularly limited, as long as it is within a range acceptable to the human body. The osmotic pressure ratio of the ophthalmic composition for contact lens of the present invention is preferably 0.5 to 5.0, more preferably 0.6 to 3.0, and particularly preferably 0.7 to 2.0. The osmotic pressure can be adjusted using an inorganic salt, polyhydric alcohol, sugar alcohol, and/or sugar, etc., according to a known method in the technical field of the present invention. The osmotic pressure ratio is the ratio of the osmotic pressure of a sample to 286 mOsm (osmotic pressure of 0.9 w/v% aqueous sodium chloride solution) based on the Japanese Pharmacopoeia, 15th revision. The osmotic pressure can be measured with reference to the osmotic measurement method (freezing point depression method) described in the Japanese Pharmacopoeia. To obtain a reference solution for measuring the osmotic pressure ratio (0.9 w/v% sodium chloride solution), sodium chloride (standard reagent according to the Japanese Pharmacopoeia) is dried for 40 to 50 minutes at 500 to 650°C, and then allowed to cool in a desiccator (silica gel). 0.900 g of the resultant is accurately measured, and the resultant is then dissolved in purified water, thus accurately preparing 100 mL of the solution. Alternatively, a commercially available reference solution for measuring the osmotic pressure ratio (0.9 w/v% aqueous sodium chloride solution) can be used.

As long as the effects of the present invention are attained, the ophthalmic composition for contact lens of the present invention may include, in addition to the aforementioned components, a suitable amount of a pharmacologically active component or a biologically active component in combination. Such components are not particularly limited, and examples include effective components in ophthalmological drugs described in Standards for Approval for the Manufacture (Import) of Non-prescription Drugs 2000 (Ippanyou Iyakuhin Seizou (Yunyuu) Syounin Kizyun 2000), edited by the Yakuji Shinsa Kenkyuukai. Specific examples of the components used in ophthalmological drugs include the following components.

Antihistamines such as iproheptine, diphenhydramine hydrochloride, chlorphenylamine maleate, ketotifen fumarate, and pemirolast potassium.

Decongestants such as tetrahydrozoline hydrochloride, naphazoline hydrochloride, naphazoline sulfate, epinephrine hydrochloride, ephedrine hydrochloride, and methylephedrine hydrochloride.

Disinfectants such as cetyl pyridinium, benzalkonium chloride, benzethonium chloride, chlorhexidine hydrochloride, chlorhexidine gluconate, and polyhexanide hydrochloride.

Vitamins such as flavin adenine dinucleotide sodium, cyanocobalamin, retinol acetate, retinol palmitate, pyridoxine hydrochloride, panthenol, calcium pantothenate, and tocopherol acetate.

Amino acids such as potassium aspartate and magnesium aspartate.

Antiphlogistics such as dipotassium glycyrrhizate, pranoprofen, allantoin, azulene, azulene sodium sulfonate, guaiazulene, berberine chloride, berberine sulfate, lysozyme chloride, and licorice.

Others, such as sodium cromoglicate, sodium chondroitin sulfate, sodium hyaluronate, sulfamethoxazole, and sodium sulfamethoxazole.

Further, as long as the effects of the invention are attained, various additives can be suitably selected by a conventional method according to the application, preparation form, etc., and added to the ophthalmic composition for contact lens of the present invention. The additives can be used singly, or in a combination in a suitable amount. Examples of the additives include those described in the Japanese Pharmaceutical Excipients Directory 2007 (edited by the International Pharmaceutical Excipients Council Japan). Typical components include the following additives.

Carriers: e.g., aqueous carriers such as water, water-containing ethanol.

Sugars: e.g., cyclodextrin.

Sugar alcohols: e.g., xylitol, sorbitol, and mannitol. These may be in the d form, l form, or dl form.

Antiseptics, disinfectants, and antibacterial agents: e.g., alkyldiaminoethylglycine hydrochloride, sodium benzoate, ethanol, benzalkonium chloride, benzethonium chloride, chlorhexidine gluconate, chloro butanol, sorbic acid, potassium sorbate, sodium dehydroacetate, methyl parahydroxybenzoate, ethyl parahydroxybenzoate, propyl parahydroxybenzoate, butyl parahydroxybenzoate, oxyquinoline sulfate, phenethyl alcohol, benzyl alcohol, and Glokill (trade name, Rhodia) .

The ophthalmic composition for contact lens of the present invention can be prepared by a method comprising adding a desired amount of each of component (A) and component (B), and if necessary, other components to a carrier in a manner such that these components have a desired concentration. For example, eye drops, solutions for wearing contact lenses, eye washes, or contact lens care solutions are prepared by dissolving or suspending the aforementioned components in purified water, adjusting the pH and osmotic pressure to the predetermined levels, and subjecting these to sterilization treatment by filter sterilization, etc. Regarding the dissolution of components (A) and (B), and the dissolution of components with a highhydrophobic property, components having an effect of helping dissolution such as surfactants may be added beforehand; then the mixture is stirred, after which purified water is added thereto, followed by dissolution or suspension.

The dosage form of the ophthalmic composition for contact lens of the present invention is not particularly limited, as long as it can be used in the field of ophthalmology. Examples of the dosage form include liquids, ointments, etc. Of these, liquids are preferable. Pharmacologically (pharmaceutically) or physiologically acceptable water in the field of medicine can be used as an aqueous carrier when the ophthalmic composition for contact lens of the present invention is liquid. Examples of such water include distilled water, water, purified water, sterile purified water, water for injection, distilled water for injection, and the like. These definitions are based on the Japanese Pharmacopoeia, 15th revision.

The preparation form of the ophthalmic composition for contact lens of the present invention is not limited, as long as it is used in the field of ophthalmology. Examples thereof include eye drops for contact lens (with examples of the eye drops including eye drops that can be used while wearing a contact lens), eye washes for contact lens (with examples of the eye washes including eye washes that can be used while wearing a contact lens), solutions for wearing a contact lens, contact lens care solutions(disinfectant solutions for contact lens, storage solutions for contact lens, cleansing solutions for contact lens, cleansing and storage solutions for contact lens, and disinfectant, cleansing and storage solutions for contact lens (multiple-purpose solutions), etc.) Of these, eye drops for contact lens are particularly preferable as the preparation form of the ophthalmic composition for contact lens of the present invention.

In the present invention, examples of the contact lens include soft contact lenses, soft contact lenses containing no water, hard contact lenses, oxygen-permeable hard contact lenses, vision corrective contact lenses, and non corrective contact lenses, and other various types of contact lenses.

Typically, a soft contact lens may mean a water- containing soft contact lens (lens having a water content of 10% or more (hereinbelow referred to as a water- containing soft contact lens) according to the vision corrective contact lens standards (hereinbelow referred to as contact lens standards) (Notification No. 349 of the Ministry of Welfare in 2001) ) ; however, in the present specification, soft contact lenses include those that do not contain water (lens having a water content less than 10% according to contact lens standards, and made of highly flexible material), in addition to the above soft contact lens.

Soft contact lenses include both of ionic contact lenses and nonionic contact lenses, and both of contact lenses with a low water content (water content: less than 50%) and contact lenses with a high water content (water content: 50% or more). That is, examples of the soft contact lens include those in Groups I to IV in SCL classification according to the U.S. Food and Drug Administration.

Soft contact lenses include both silicone hydrogel contact lenses (hereinafter referred to as SHCL) and non-silicone hydrogel contact lenses (soft contact lenses other than silicone hydrogel lenses). The silicone hydrogel contact lenses include both ionic silicone hydrogel contact lenses and nonionic silicone hydrogel contact lenses.

In particular, bacterial adhesion to a contact lens easily occurs in soft contact lenses (particularly, water-containing soft contact lenses), and inhibition of bacterial adhesion to soft contact lenses is strongly desired. Thus, as the preparation form of the ophthalmic composition for contact lens of the present invention, an ophthalmic composition for contact lens which is an ophthalmic composition for soft contact lens (i.e., the lens to be applied is a soft contact lens, in particular, a water-containing soft contact lens), is preferable.

Examples of the soft contact lens include those containing, as a constituent material, a polymer that contains as a polymerization component one or more monomers selected from 2-hydroxyethyl methacrylate (HEMA), 2,3-dihydroxypropyl methacrylate (i.e., glycerol methacrylate), N-vinyl pyrrolidone, methacrylic acid and the sodium salt thereof, polyvinyl alcohol, n-butyl methacrylate, and n-butyl acrylate.

Further, bacteria adhesion and/or lipid adsorption easily occur particularly on silicon hydrogel lenses; thus, the inhibition of bacterial adhesion and/or lipid adsorption on silicone hydrogel lenses is strongly desired.

Thus, an ophthalmic composition for contact lens, whose target lens is a silicone hydrogel lens, is particularly preferable.

Typically, a hard contact lens may mean a non- oxygen- permeable hard contact lens; however, in the present specification, the hard contact lens includes an oxygen- permeable hard contact lens in addition to the non- oxygen-permeable hard contact lens, unless otherwise specified.

Examples of the hard contact lens include those containing, as a constituent material, a polymer that contains as a polymerization component, one or more monomers selected from methyl methacrylate, cellulose acetate butyrate, tris(trimethylsiloxy)silyl propyl methacrylate, pentamethyl disiloxanyl propyl methacrylate, trifluoroethylmethacrylate, hexafluoro isopropyl methacrylate, and perfluorooctylethyl methacrylate.

The "water content" of the contact lens means the weight proportion of water contained in the lens based on the weight of the entire lens.

As the container that holds the ophthalmic composition for contact lens of the present invention, a container that can typically be used to hold an ophthalmic composition for contact lens can be used. The container may be made of glass or plastic. When a plastic container is used to hold the ophthalmic composition for contact lens of the present invention, examples of constituent materials of the plastic container include, but are not particularly limited to, polyethylenenaphthalate, polyarylate, polyethylene terephthalate, polypropylene, polyethylene, polyimide, a copolymer thereof, and a mixture of two or more of them. Examples of the copolymers include those that contain another polyester unit and imide unit, in addition to any one of ethylene- 2, 6- naphthalate units, arylate units, ethylene terephthalate units, propylene units, ethylene units, and imide units as a main component. A particularly preferable example thereof is polyethylene terephthalate.

The ophthalmic composition for contact lens of the present invention inhibits bacterial adhesion to a contact lens, and prevents bacterial infectious diseases of ocular- mucous membrane. Thus, the ophthalmic composition for contact lens of the present invention can be used for preventing bacterial infectious diseases of ocular- mucous membrane. The ophthalmic composition for contact lens of the present invention can inhibit lipid adsorption to a contact lens, and thereby inhibit blurry vision and a foreign body sensation due to lipid deposits on the contact lens. Thus, the ophthalmic composition of the present invention can be used for reducing discomfort during the wearing of a contact lens. Lipids, the adsorption of which is to be inhibited, are preferably those originating from bodily secretions or cosmetics. Examples of such lipids include simple lipids such as fats (triacylglycerol), waxes (fatty acid esters of higher alcohols), sterol esters, fatty acid esters of vitamins; complex lipids such as glycerophospholipids, sphingophospholipids, glyceroglycolipids, sphingoglycolipids, lipids with C- P binding, and sulfolipids; and derived lipids such as steroid (e.g., cholesterol), fatty acids, higher alcohols, fat- soluble vitamins, and hydrocarbons. In particular, derived lipids are preferable, and cholesterol is most preferable.

According to the application and preparation form of the composition, the ophthalmic composition for contact lens of the present invention can be used in the same manner as in the conventional ophthalmic compositions for contact lens.

Specifically, for example, when the ophthalmic composition for contact lens of the present invention is used as eye drops for contact lens, it can be instilled into eyes during or before the wearing of the contact lens, as with conventional eye drops. When the ophthalmic composition for contact lens of the present invention is used as an eye wash for contact lens, eyes can be washed using the ophthalmic composition during or before the wearing of the contact lens, as with conventional eye washes.

Alternatively, when the ophthalmic composition for contact lens of the present invention is used as a multipurpose solution, a contact lens can be washed, rinsed, and then stored using the ophthalmic composition of the present invention, as with conventional multipurpose solutions.

During such use, the ophthalmic composition for contact lens of the present invention is brought into contact with the contact lens.

2. Method for Inhibiting bacterial adhesion to Contact Lens, and Method for Providing Ophthalmic Composition for Contact Lens with Effect of Inhibiting bacterial adhesion to Contact Lens

As described above, the combination use of components (A) and (B) can inhibit bacterial adhesion to a contact lens.

Thus, from a different viewpoint, the present invention provides a method for inhibiting bacterial adhesion to a contact lens, comprising bringing an ophthalmic composition for contact lens containing (A) polyoxyethylene castor oil and (B) terpenoid into contact with the contact lens.

The present invention also provides a method for providing an ophthalmic composition for contact lens with an effect of inhibiting bacterial adhesion to a contact lens, comprising adding (A) polyoxyethylene castor oil and (B) terpenoid to the ophthalmic composition for contact lens.

In these methods, the kinds of component (A) and component (B) to be used, the contents and the ratio thereof, the kinds and the contents of components added other than the above, the preparation form of the ophthalmic composition for contact lens, the kind of contact lens, the container, the embodiment method, and the like, are the same as in the "1. Ophthalmic Composition for Contact Lens" section above.

In particular, these methods are suitably applied to soft contact lenses, particularly those belonging to Group IV. The methods are also suitably applied to silicone hydrogel lenses.

### 3. Method for Inhibiting Lipid Adsorption to Contact Lens and Method for Providing Ophthalmic Composition for Contact Lens with Effect of Reducing Lipid Adsorption to Contact Lens

As described above, the combination use of components (A) and (B) can inhibit bacterial adhesion to a contact lens.

Thus, from another different viewpoint, the present invention provides a method for inhibiting lipid adsorption to a contact lens, comprising bringing an ophthalmic composition for contact lens containing (A) polyoxyethylene castor oil and (B) terpenoid into contact with the contact lens.

The present invention further provides a method for providing an ophthalmic composition for contact lens with an effect of inhibiting lipid adsorption to a contact lens, comprising adding (A) polyoxyethylene castor oil and (B) terpenoid to the ophthalmic composition for contact lens.

In these methods, the kinds of component (A) and component (B) to be used, the contents and the ratio thereof, the kinds and the contents of components added other than the above, the preparation form of the ophthalmic composition for contact lens, the kind of contact lens, the container, the embodiment method, and the like, are the same as in or according to the "1. Ophthalmic Composition for Contact Lens" section above.

In particular, these methods are suitably applied to hard contact lenses. The methods are also suitably applied to silicone hydrogel lenses.

Examples

The present invention is explained in detail below based on the Examples; however, the present invention is not limited to these Examples.

### Test Example 1: Test for Measuring bacterial adhesion to Contact Lens (1)

Test liquids having the formulations shown in Table 1 (Examples 1-1 and 1-2, and Comparative Examples 1-1 to 1-4) were prepared by a standard method, and adhesion of bacteria, *Pseudomonas aeruginosa* (ATCC9027) to soft contact lenses (SCLs) (SCL classification according to the U.S. Food and Drug Administration: Group IV; main material: "etafilcon A") was evaluated using these liquids. In the preparation of the test liquids, borax was added in an amount such that the pH of each test liquid became 7 (the same hereinafter).

Each of the lenses was immersed in 5 mL of sterilized physiological saline for 4 hours or more (lens pretreatment). 1 mL of each test liquid was individually placed in an individual well of a 24-well multiplate. Water on the surface of each lens was slightly drained at the edge of the plate, and the lenses were placed in individual wells. Physiological saline was used as a control (n=3). 24 hours later, water of each lens was slightly drained at the edge of the plate, and then the lenses were placed in individual wells of a 12-well multiplate containing 5 ml of *Pseudomonas aeruginosa* liquid ($10^7$ to $10^8$ CFU/mL) (suspended in physiological saline) per well. The lenses were stored at room temperature for 30 minutes. Subsequently, each lens was placed using tweezers in individual wells of a 6-well plate containing 5 mL of physiological saline per well, and the plate was shaken for one minute. The lenses were transferred to individual Spitz tubes each containing 5 mL of another physiological saline, and subjected to ultrasonic waves (38 kHz) for 3 minutes, followed by stirring for one minute with a tube mixer. Thus, bacteria adhered to each SCL were removed to form bacterial adhesion liquids.

Each of the obtained bacterial adhesion liquids was diluted to a concentration appropriate for the measurement, seeded in a soybean-casein digest agar medium (SCDLP agar medium), and cultured overnight at 33°C. Thereafter, the number of observed colonies was counted, and corrected by the dilution ratio to determine the cell count (viable cell count) adhered to each lens. The bacterial adhesion inhibition rate (%) relative to the adhesion cell count obtained using the test liquid of Comparative Example 1-1 (control) was calculated according to the following formula.

Math. 1

bacterial adhesion inhibition rate (%) = {1- (adhesion cell count of each test liquid / adhesion cell count of test liquid of Comparative Example 1-1)} x 100

Table 1 shows the obtained results. The use of polyoxyethylene castor oil alone (Comparative Examples 1-2 and 1-3) tended to show an equivalent or rather lower bacterial adhesion inhibition rate (i.e., increased bacterial adhesion) to or than the control (Comparative Example 1-1), and showed no bacterial adhesion inhibition effect. Additionally, the use of menthol alone (Comparative Example 1-4) showed almost no bacterial adhesion inhibition effect. In contrast, the combination use of polyoxyethylene castor oil and menthol (Examples 1-1 and 1-2) unexpectedly showed an increased excellent bacterial adhesion inhibition effect compared to the use of menthol alone.

Table 1

| Unit (w/v%) | | | | | | |
|---|---|---|---|---|---|---|
| | Comparative Example 1-1 | Comparative Example 1-2 | Comparative Example 1-3 | Comparative Example 1-4 | Example 1-1 | Example 1-2 |
| Polyoxyethylene castor oil 10 | - | - | 0.25 | - | - | 0.25 |
| Polyoxyethylene castor oil 35 | - | 0.25 | 0.25 | - | 0.25 | 0.25 |
| l-menthol | - | - | - | 0.015 | 0.015 | 0.015 |
| Boric acid | 0.2 | 0.2 | 0.2 | 0.2 | 0.2 | 0.2 |
| Borax | q.s. | q.s. | q.s. | q.s. | q.s. | q.s. |
| Purified water | Balance | Balance | Balance | Balance | Balance | Balance |
| Total amount | 100 mL | 100 mL | 100 mL | 100 mL | 100 mL | 100 mL |
| pH | 7 | 7 | 7 | 7 | 7 | 7 |
| bacterial adhesion inhibition rate (%) | - | -6.7 | -1.5 | 2.7 | 9.2 | 27.2 |

Test Example 2: Test for Measuring bacterial adhesion to Contact Lens (2)

A test for measuring bacterial adhesion was performed using the test liquids shown in Table 1 (Comparative Example 1-1) and Table 2 (Examples 1-3 and 1-4, and Comparative Examples 1-5 and 1-6) in the same manner as in Test Example 1. The bacterial adhesion inhibition rate (%) relative to the adhesion cell count obtained using the test liquid of Comparative Example 1-1 (control) was calculated.

Table 2 shows the obtained results. The use of camphor alone (Comparative Example 1-5) or borneol alone (Comparative Example 1-6) showed almost no bacterial adhesion inhibition effect, or showed rather a decreased bacterial adhesion inhibition rate (increased bacterial adhesion) compared to the control (Comparative Example 1-1). In contrast, the combination use of polyoxyethylene castor oil and camphor or borneol (Example 1-3 or 1-4) unexpectedly showed an excellent bacterial adhesion inhibition effect.

Table 2

| Unit (w/v%) | | | | |
|---|---|---|---|---|
| | Comparative Example 1-5 | Comparative Example 1-6 | Example 1-3 | Example 1-4 |
| Polyoxyethylene castor oil 10 | - | - | 0.25 | 0.25 |
| Polyoxyethylene castor oil 35 | - | - | 0.25 | 0.25 |
| d-camphor | 0.005 | - | 0.005 | - |
| d-borneol | - | 0.005 | - | 0.005 |
| Boric acid | 0.2 | 0.2 | 0.2 | 0.2 |
| Borax | q.s. | q.s. | q.s. | q.s. |

(continued)

| Unit (w/v%) | | | | |
|---|---|---|---|---|
| | Comparative Example 1-5 | Comparative Example 1-6 | Example 1-3 | Example 1-4 |
| Purified water | Balance | Balance | Balance | Balance |
| Total amount | 100 mL | 100 mL | 100 mL | 100 mL |
| pH | 7 | 7 | 7 | 7 |
| bacterial adhesion inhibition rate (%) | -1.0 | -21.1 | 16.1 | 11.5 |

Reference Test 1: Test for Measuring bacterial adhesion to Contact Lens (3)

A test for measuring bacterial adhesion was performed using the test liquids shown in Table 1 (Comparative Example 1-1) and Table 3 (Reference Examples 1-1 to 1-3) in the same manner as in Test Example 1. The bacterial adhesion inhibition rate (%) relative to the adhesion cell count obtained using the test liquid of Comparative Example 1-1 (control) was calculated.

Table 3 shows the obtained results. Although the use of polyoxyethylene hydrogenated castor oil alone (Reference Example 1-1) showed a bacterial adhesion inhibition effect, the combination use of polyoxyethylene hydrogenated castor oil and menthol (Reference Example 1-2), and the combination use of polyoxyethylene hydrogenated castor oil and camphor (Reference Example 1-3) rather showed increased bacterial adhesion to the contact lens compared to Reference Example 1-1. This indicates that unlike polyoxyethylene castor oil, polyoxyethylene hydrogenated castor oil, which is a nonionic surfactant like polyoxyethylene castor oil, does not show an increased bacterial adhesion inhibition effect when used in combination with terpenoid.

Table 3

| Unit (w/v%) | | | |
|---|---|---|---|
| | Reference Example 1-1 | Reference Example 1-2 | Reference Example 1-3 |
| Polyoxyethylene hydrogenated castor oil 60 | 0.5 | 0.5 | 0.5 |
| 1-menthol | - | 0.015 | - |
| d-camphor | - | - | 0.005 |
| Boric acid | 0.2 | 0.2 | 0.2 |
| Borax | q.s. | q.s. | q.s. |
| Purified water | Balance | Balance | Balance |
| Total amount | 100 mL | 100 mL | 100 mL |
| pH | 7 | 7 | 7 |
| bacterial adhesion inhibition rate (%) | 31.1 | 27.4 | 2.6 |

The results surprisingly reveal that although terpenoid does not always exhibit a bacterial adhesion inhibition effect, and polyoxyethylene castor oil exhibits no bacterial adhesion inhibition effect, the combination use of terpenoid and polyoxyethylene castor oil exhibits an excellent bacterial adhesion inhibition effect.

This effect was not attained when polyoxyethylene hydrogenated castor oil was used in place of polyoxyethylene castor oil.

Test Example 3: Test for Measuring Lipid Adsorption to PMMA (1)

Lipid adsorption to hard contact lenses were evaluated using the test liquids (Examples 2-1 and 2-2, and Comparative Examples 2-1 to 2-4) shown in Table 4. As a hard contact lens, a chip obtained by uniformly cutting a polymethylmethacrylate (PMMA) resin, which is a material of hard contact lens, into a circular shape, was used.

1, 000 μL of each test liquid (Examples 2- 1 and 2- 2, and Comparative Examples 2- 1 to 2- 4) was individually placed in an individual well of a 24- well microplate, and a PMMA chip was immersed in each well, followed by shaking at 34°C for 24 hours. After completion of the shaking, each PMMA chip was rinsed in about 200 mL of a physiological salt solution, and transferred to a 24- well microplate containing 1, 000 μL of a fluorescence lipid solution per well. Shaking was then conducted again at 34°C for 24 hours. 24 hours later, PMMA that had undergone shaking treatment was transferred to a plate containing 1 mL of another physiological saline, and the fluorescence intensity of each well was measured using a fluorescence plate leader (excitation wavelength: 485 nm, fluorescence wavelength: 538 nm) . As a fluorescence lipid solution, 4 ml of 2% polysorbate was added to 500 μL of a mixture in which a chloroform solution (1 mg/ml) of  fluorescent- labeled lipids (cholesterol, 25- NBD- (powder) ; non- cholesterol, 25- {N- [(7- nitro- 2, 1, 3- Benzoxadiazol- 4- yl)- methyl] amino}- 27; both produced by Avanti Polar Lipids, Co., Ltd.) and methanol were contained in a volume ratio of 1: 4. Stirring was performed, and physiological saline (0.9 w/v% sodium chloride) was added to the mixture. Thus, a mixture having a total amount of 20 ml was used.

The lipid adsorption inhibition rate of each test liquid relative to the lipid adsorption amount of the control (Comparative Example 2-1) was calculated according to the following formula.

Math. 2

Lipid adsorption inhibition rate (%) = {1- (fluorescent intensity of each test liquid / fluorescent intensity of Comparative Example 2-1)} x 100

Table 4 shows the results. The use of polyoxyethylene castor oil alone (Comparative Examples 2-2 and 2-3) showed almost the same or slightly higher lipid adhesion inhibition effect as or than the control (Comparative Example 2-1); and the menthol alone (Comparative Example 2-4) had a noticeably lowered lipid adhesion inhibition rate (i.e., increased lipid adhesion). In contrast, the combination use of polyoxyethylene castor oil and menthol (Example 2-1 or 2-2) unexpectedly showed an excellent lipid adsorption inhibition effect.

Table 4

| Unit (w/v%) | | | | | | |
|---|---|---|---|---|---|---|
| | Comparative Example 2-1 | Comparative Example 2-2 | Comparative Example 2-3 | Comparative Example 2-4 | Example 2-1 | Example 2-2 |
| Polyoxyethylene castor oil 3 | - | - | 0.25 | - | - | 0.25 |
| Polyoxyethylene castor oil 35 | - | 0.25 | 0.25 | - | 0.25 | 0.25 |
| l-menthol | - | - | - | 0.015 | 0.015 | 0.015 |
| Boric acid | 0.2 | 0.2 | 0.2 | 0.2 | 0.2 | 0.2 |
| Borax | q.s. | q.s. | q.s. | q.s. | q.s. | q.s. |
| Purified water | Balance | Balance | Balance | Balance | Balance | Balance |
| Total amount | 100 mL | 100 mL | 100 mL | 100 mL | 100 mL | 100 mL |
| pH | 7 | 7 | 7 | 7 | 7 | 7 |
| bacterial adhesion inhibition rate (%) | - | -0.2 | 6.4 | -22.8 | 8.2 | 20.1 |

Reference Test Example 2: Test for Measuring Inhibition of Lipid Adsorption to PMMA (2)

A test for measuring inhibition of lipid adsorption was performed using test liquids shown in Table 4 (Comparative Example 2-1) and Table 5 (Reference Examples 2-1 and 2-2) in the same manner as in Test Example 3. The lipid adsorption inhibition rate (%) relative to the lipid adsorption amount of the control (Comparative Example 2-1) was calculated.

Table 5 shows the obtained results. Although polyoxyethylene hydrogenated castor oil alone (Reference Example 2-1) showed a lipid adsorption inhibition effect, the combination use of polyoxyethylene hydrogenated castor oil and menthol (Reference Example 2-2) rather had more lipid adsorption to a contact lens than Reference 2-1. This indicates that polyoxyethylene hydrogenated castor oil, which is a nonionic surfactant like polyoxyethylene castor oil, does not show an increased lipid adsorption inhibition effect when used in combination with terpenoid.

Table 5

| Unit (w/v%) | | |
|---|---|---|
| | Reference Example 2-1 | Reference Example 2-2 |
| Polyoxyethylene hydrogenated castor oil 60 | 0.5 | 0.5 |
| l-menthol | - | 0.015 |
| Boric acid | 0.2 | 0.2 |
| Borax | q.s. | q.s. |
| Purified water | Balance | Balance |
| Total amount | 100 mL | 100 mL |
| pH | 7 | 7 |
| Lipid adsorption inhibition rate (%) | 17.4 | 10.0 |

The results surprisingly reveal that although polyoxyethylene castor oil exhibited almost no lipid adhesion inhibition effect, and terpenoid rather increased lipid adhesion, the combination use of polyoxyethylene castor oil and terpenoid exhibited an excellent lipid adhesion inhibition effect. In contrast, the combination use of terpenoid with polyoxyethylene hydrogenated castor oil, which was used in place of polyoxyethylene castor oil, rather reduced the lipid adhesion inhibition effect.

Test Example 4: Test for Measuring bacterial adhesion to Contact Lens (4)

Test liquids shown in Tables 6 and 7 (Examples 3a-1 to 3a-4 and 3b-1 to 3b-4, and Comparative Examples 3a and 3b) were prepared according to a standard method. Using these liquids, a test for measuring bacterial adhesion to a contact lens was performed in the same manner as in Test Example 1.

Tables 6 and 7 show the results. Examples 3a-1 to 3a-4 in which one or two materials selected from menthol, geraniol, and eucalyptus oil was contained in addition to polyoxyethylene castor oil 10 showed a noticeably higher bacterial adhesion inhibition effect than Comparative Example 3a containing polyoxyethylene castor oil 10 alone. Similarly, Examples 3b-1 to 3b-5 containing menthol, geraniol, and eucalyptus oil showed a noticeably higher bacterial adhesion inhibition effect than Comparative Example 3b containing polyoxyethylene castor oil 35 alone. As eucalyptus oil, a product having a cineole content of 80 w/v% or more according to the Japanese Pharmacopoeia, 15th revision, was used.

The results reveal that the combination use of polyoxyethylene castor oil and terpenoid showed a noticeably high bacterial adhesion inhibition effect in the ophthalmic composition for contact lens

Table 6

| Unit (w/v%) | | | | | |
|---|---|---|---|---|---|
| | Comparative Example 3a | Example 3a-1 | Example 3a-2 | Example 3a-3 | Example 3a-4 |
| Polyoxyethylene castor oil 10 | 0.05 | 0.05 | 0.05 | 0.05 | 0.05 |
| l-menthol | - | 0.005 | - | - | 0.005 |
| Geraniol | - | - | 0.005 | - | - |
| Eucalyptus oil | - | - | - | 0.005 | 0.005 |
| Polyoxyethylene hydrogenated castor oil 60 | 0.05 | 0.05 | 0.05 | 0.05 | 0.05 |
| Boric acid | 0.2 | 0.2 | 0.2 | 0.2 | 0.2 |
| Borax | q.s. | q.s. | q.s. | q.s. | q.s. |
| Purified water (mL) | Balance | Balance | Balance | Balance | Balance |
| pH | 7 | 7 | 7 | 7 | 7 |
| bacterial adhesion inhibition rate (%) | - | 13.8 | 25.9 | 36.1 | 26.3 |

Table 7

| Unit (w/v%) | | | | | | |
|---|---|---|---|---|---|---|
| | Comparative Example 3b | Example 3b-1 | Example 3b-2 | Example 3b-3 | Example 3b-4 | Example 3b-5 |
| Polyoxyethylene castor oil 35 | 0.05 | 0.05 | 0.05 | 0.05 | 0.05 | 0.05 |
| l-menthol | - | 0.005 | - | - | 0.005 | 0.005 |
| Geraniol | - | - | 0.005 | - | 0.005 | - |
| Eucalyptus oil | - | - | - | 0.005 | - | 0.005 |
| Polyoxyethylene hydrogenated castor oil 60 | 0.05 | 0.05 | 0.05 | 0.05 | 0.05 | 0.05 |
| Boric acid | 0.2 | 0.2 | 0.2 | 0.2 | 0.2 | 0.2 |
| Borax | q.s. | q.s. | q.s. | q.s. | q.s. | q.s. |
| Purified water (mL) | Balance | Balance | Balance | Balance | Balance | Balance |
| pH | 7 | 7 | 7 | 7 | 7 | 7 |
| bacterial adhesion inhibition rate (%) | - | 8.77 | 11.1 | 24.6 | 26.2 | 39.9 |

Test Example 5 Test for Measuring bacterial adhesion to Contact Lens (5)

Test liquids shown in the Table below (Examples 3c-1 and 3c-2, and Comparative Examples 3c-1 and 3c-2) were prepared according to a standard method. Using these test liquids, a test for measuring bacterial adhesion to a contact lens was performed in the same manner as in Test Example 1.

Table 8 shows the results. Example 3c-1 containing menthol, geraniol and eucalyptus oil in addition to polyoxyethylene castor oil 10 showed a noticeably higher bacterial adhesion inhibition effect than Comparative Example 3c-1 containing polyoxyethylene castor oil 10 alone. Similarly, Example 3c-2 containing menthol and geraniol in addition to polyoxyethylene castor oil 10 and polyoxyethylene castor oil 35 showed a noticeably higher bacterial adhesion inhibition effect than Comparative Example 3c-2 containing polyoxyethylene castor oil 10 and polyoxyethylene castor oil 35 alone.

The results reveal that the combination use of polyoxyethylene castor oil and terpenoid in the ophthalmic composition for contact lens exhibits a noticeably high bacterial adhesion inhibition effect

Table 8

| Unit (w/v%) | | | | |
|---|---|---|---|---|
| | Comparative Example 3c-1 | Example 3c-1 | Comparative Example 3c-2 | Example 3c-2 |
| Polyoxyethylene castor oil 10 | 0.5 | 0.5 | 0.5 | 0.5 |
| Polyoxyethylene castor oil 35 | - | - | 0.25 | 0.25 |
| l-menthol | - | 0.005 | - | 0.005 |
| Geraniol | - | 0.005 | - | 0.005 |
| Eucalyptus oil | - | 0.005 | - | - |
| Polysorbate 80 | 0.25 | 0.25 | - | - |
| Boric acid | 0.2 | 0.2 | 0.2 | 0.2 |
| Borax | q.s. | q.s. | q.s. | q.s. |
| Purified water (mL) | Balance | Balance | Balance | Balance |
| pH | 7 | 7 | 7 | 7 |
| bacterial adhesion inhibition rate (%) | - | 12.3 | - | 21.7 |

Preparation Examples

According to the formulations shown in Table 9, eye drops for contact lens (Preparation Examples 1 to 7), an eye wash for contact lens (Preparation Example 8), a solution for wearing a contact lens (Preparation Example 9), and a multiple-purpose solution for contact lens (Preparation Example 10) were prepared by a standard method.
The unit of the osmotic pressure is mOsm (milliosmol).

Table 9

| Unit: g/100 mL | Preparation Example | | | | | | | | | |
|---|---|---|---|---|---|---|---|---|---|---|
| | 1 | 2 | 3 | 4 | 5 | 6 | 7 | 8 | 9 | 10 |
| Polyoxyethylene castor oil 35 | 0.050 | | 0.400 | | | 0.500 | 0.200 | | | |
| Polyoxyethylene castor oil 10 | 0.100 | 0.050 | 0.100 | 0.500 | 0.350 | 0.010 | 0.020 | 0.100 | 0.050 | 0.100 |
| Polyoxyethylene castor oil 3 | | | 0.500 | 0.085 | | | | | | 0.100 |
| l-menthol | 0.010 | | 0.040 | 0.015 | 0.020 | 0.030 | 0.050 | 0.002 | 0.025 | 0.010 |
| d-camphor | 0.005 | | 0.010 | | | | | | | |
| d-borneol | 0.003 | | | 0.015 | 0.001 | | | 0.001 | | |

(continued)

| Unit: g/100 mL | Preparation Example | | | | | | | | | |
|---|---|---|---|---|---|---|---|---|---|---|
| | 1 | 2 | 3 | 4 | 5 | 6 | 7 | 8 | 9 | 10 |
| Geraniol | | 0.005 | 0.003 | | 0.001 | | 0.002 | | | |
| Neostigmine methylsulfate | | 0.010 | | | | | | | | |
| Tetrahydrozoline hydrochloride | 0.010 | | | | | | | | | |
| Chlorpheniramine maleate | 0.030 | 0.015 | 0.010 | | 0.030 | | | | | |
| Azulene sodium sulfonate | | | | | 0.020 | | | | | |
| Berberine sulfate | | | | | 0.005 | | | | | |
| Dipotassium glycyrrhizinate | 0.250 | | | | | | | | 0.025 | |
| Pyridoxine hydrochloride | 0.100 | 0.100 | 0.050 | | | | | | 0.010 | |
| Tocopherol acetate | | | | | | | | | 0.005 | |
| Aminoethylsulfonic acid | | | 0.500 | | | | | | 0.020 | |
| Sodium chondroitin sulfate | 0.100 | 0.500 | 0.500 | | 0.500 | | 0.500 | | 0.040 | |
| Sodium edetate | 0.010 | 0.010 | | | 0.050 | | 0.004 | | 0.050 | |
| Sodium chloride | | | | 0.450 | | 0.400 | 0.300 | 0.450 | | 0.700 |
| Potassium chloride | | | | 0.080 | | 0.080 | 0.100 | 0.100 | | 0.050 |
| Disodium hydrogen phosphate | | | | | | | | 0.150 | | 0.150 |
| Sodium dihydrogen phosphate | | | | | | | | 0.050 | | 0.200 |
| Boric acid | 1.500 | 1.800 | 1.000 | | 1.500 | 1.000 | 1.100 | | 1.200 | |
| Borax | 0.500 | 0.350 | 0.050 | | 0.300 | 0.200 | 0.300 | | 0.300 | |
| Hydroxyethyl cellulose | | | | 0.250 | | 0.050 | | | | |
| Hydroxypropyl methyl cellulose | | | | 0.250 | | | | 0.100 | 0.300 | |
| Glycerin | | | | 0.100 | | | | | | |
| Poloxamer 407 | | 0.100 | | | | 0.050 | | | | |
| Polysorbate 80 | 0.500 | | 0.300 | 0.500 | 0.200 | | | 0.300 | 0.500 | |
| Polyoxyethylene hydrogenated castor oil 60 | | 0.200 | | | 0.500 | 0.300 | 0.300 | | | |
| Potassium sorbate | | 0.050 | | | | 0.050 | 0.080 | | | |
| Polyhexamethylene biguanide | | | | | | | | | | 0.0001 |

(continued)

| Unit: g/100 mL | Preparation Example | | | | | | | | | |
|---|---|---|---|---|---|---|---|---|---|---|
| | 1 | 2 | 3 | 4 | 5 | 6 | 7 | 8 | 9 | 10 |
| Dibutyl hydroxytoluene | | | | | 0.001 | | | | | |
| Chlorobutanol | | | 0.150 | | | | | | | |
| Ethanol | - | | | | | 0.100 | | | | |
| Hydrochloric acid | q.s. | q.s. | q.s. | q.s. | q.s. | q.s. | q.s. | q.s. | q.s. | q.s. |
| Sodium hydroxide | q.s. | q.s. | q.s. | q.s. | q.s. | q.s. | q.s. | q.s. | q.s. | q.s. |
| Purified water | q.s. | q.s. | q.s. | q.s. | q.s. | q.s. | q.s. | q.s. | q.s. | q.s. |
| Total content | 100 mL | 100 mL | 100 mL | 100 mL | 100 mL | 100 mL | 100 mL | 100 mL | 100 mL | 100 mL |
| pH | 7.0 | 7.0 | 5.8 | 7.5 | 7.0 | 7.4 | 7.5 | 6.2 | 7.0 | 7.5 |
| Osmotic pressure | 370 | 300 | 300 | 360 | 350 | 350 | 360 | 300 | 350 | 290 |

**Claims**

1.  An ophthalmic composition for contact lens, comprising (A) polyoxyethylene castor oil and (B) terpenoid.

2.  The ophthalmic composition for contact lens according to claim 1, wherein the total content of component (B) is contained in a total amount of 0.01 to 1,000 parts by weight per 100 parts by weight of the total amount of component (A).

3.  The ophthalmic composition for contact lens according to claim 1 or 2, which further comprises a buffer.

4.  The ophthalmic composition for contact lens according to any one of claims 1 to 3, which further comprises a nonionic surfactant other than polyoxyethylene castor oil.

5.  A method for inhibiting bacterial adhesion to a contact lens, comprising bringing an ophthalmic composition for contact lens containing (A) polyoxyethylene castor oil and (B) terpenoid into contact with the contact lens.

| **INTERNATIONAL SEARCH REPORT** | International application No. |
|---|---|
| | PCT/JP2011/079064 |

**A. CLASSIFICATION OF SUBJECT MATTER**
*A61K9/08*(2006.01)i, *A61K31/045*(2006.01)i, *A61K31/122*(2006.01)i,
*A61K31/765*(2006.01)i, *A61L2/18*(2006.01)i, *A61P31/04*(2006.01)i, *G02C13/00*
(2006.01)i
According to International Patent Classification (IPC) or to both national classification and IPC

**B. FIELDS SEARCHED**

Minimum documentation searched (classification system followed by classification symbols)
A61K9/08, A61K31/045, A61K31/122, A61K31/765, A61L2/18, A61P31/04,
G02C13/00

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2006-321790 A (Taisho Pharmaceutical Co., Ltd.), 30 November 2006 (30.11.2006), claims; paragraph [0017]; example 4 (Family: none) | 1-5 |
| A | JP 2008-222638 A (Teika Pharmaceutical Co., Ltd.), 25 September 2008 (25.09.2008), entire text (Family: none) | 1-5 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 January, 2012 (04.01.12) | 17 January, 2012 (17.01.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2011/079064 |

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/132342 A1  (Santen Pharmaceutical Co., Ltd.), 14 December 2006 (14.12.2006), entire text & JP 2007-16024 A  & US 2009/0209599 A1 & EP 1889620 A1  & CA 2610881 A & NO 20080158 A  & KR 10-2008-0029974 A & CN 101193634 A | 1-5 |
| A | JP 2002-356420 A  (Santen Pharmaceutical Co., Ltd.), 13 December 2002 (13.12.2002), entire text & WO 2002/076442 A1 | 1-5 |

Form PCT/ISA/210 (continuation of second sheet) (July 2009)

**REFERENCES CITED IN THE DESCRIPTION**

**Patent documents cited in the description**

- JP 2003248200 A
- JP 2009533081 A
- JP 2005298448 A
- JP 2004315517 A

**Non-patent literature cited in the description**

- Standards for Approval for the Manufacture (Import) of Non-prescription Drugs 2000
- Japanese Pharmaceutical Excipients Directory. 2007